# EUROPÄISCHE PATENTSCHRIFT

(11) **EP 0 982 314 B1**
(45) Veröffentlichungstag und Bekanntmachung des Hinweises auf die Patenterteilung: **07.05.2003**
(21) Anmeldenummer: 99116208.2
(22) Anmeldetag: 17.08.1999
(51) Int. Cl.: C07F 9/6568, C07F 9/6571, C07C 45/50

(54) **Valeraldehyd und Verfahren zu seiner Herstellung**
Valeraldehyde and process for its preparation
Valeraldehyde et procédé pour sa préparation

(30) Priorität: 26.08.1998 DE 19838742
(43) Veröffentlichungstag der Anmeldung: 01.03.2000
(73) Patentinhaber: Celanese Chemicals Europe GmbH, 60439 Frankfurt am Main (DE)
(72) Erfinder: Geissler, Holger, Dr., 55116 Mainz (DE); van Leeuwen, Petrus W.N.M., Prof., 3628 CA Kockengen (NL); Kamer, Paul C.J., 1412 NW Naarden (NL); van der Veen, Lars A., 1058 KW, Amsterdam (NL)

(56) Entgegenhaltungen:
- EP-A- 0 213 639
- EP-A- 0 530 015
- WO-A-95/30680
- WO-A-97/33854
- HOBBS C.F. ET AL.: JOURNAL OF ORGANIC CHEMISTRY., Bd. 46, Nr. 22, 23. Oktober 1981 (1981-10-23), Seiten 4422-4427, XP002155413 AMERICAN CHEMICAL SOCIETY. EASTON., US ISSN: 0022-3263
- VAN DER VEEN LARS A. ET AL.: "Hydroformylation of internal olefins to linear aldehydes with novel Rhodium catalysts" ANGEWANDTE CHEMIE. INTERNATIONAL EDITION., Bd. 38, Nr. 3, 1. Februar 1999 (1999-02-01), Seiten 336-338, XP000960496 VERLAG CHEMIE. WEINHEIM., DE ISSN: 0570-0833
- VAN DER VEEN LARS A. ET AL.: "New phosphacyclic diphosphines for Rhodium- catalyzed hydroformylation" ORGANOMETALLICS., Bd. 18, Nr. 23, Oktober 1999 (1999-10), Seiten 4765-4777, XP000964505 ACS, COLUMBUS, OH., US ISSN: 0276-7333

## Beschreibung

Die vorliegende Erfindung betrifft zweizähnige Phosphinliganden sowie ein Verfahren zur Herstellung linearer Aldehyde durch Hydroformylierung interner Olefine unter Verwendung solcher Phosphinliganden.

Lineare Aldehyde, insbesondere Butyraldehyd, sind von großer industrieller Bedeutung und finden nach der Weiterverarbeitung zu den Alkoholen breite Anwendung im Bereich der Weichmacher, der Lösemittel und der Polymere. Da Gemische von internen Olefinen, wie z.B. Raffinat-II, in großen Mengen als Nebenprodukt bei der Raffinierung und dem Cracking in der ölverarbeitenden Industrie anfällt, ist die Hydroformylierung von internen Olefinen zu linearen Aldehyden von großem industriellen Interesse.

Unter dem Begriff "interne Olefine" werden solche Olefine verstanden, die mindestens eine nicht endständige Doppelbindung aufweisen. Dies bedeutet aber nicht, daß interne Olefine keine endständige Doppelbindung aufweisen dürfen. Demzufolge wird unter dem Begriff "internes Olefin" beispielsweise auch eine Verbindung wie 1,3-Pentadien verstanden.

Es ist bekannt, Aldehyde durch Hydroformylierung von Olefinen unter Verwendung eines Katalysators herzustellen, wobei in der Regel nebeneinander lineare und verzweigte Aldehyde gebildet werden:

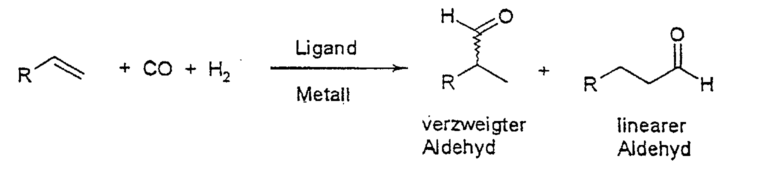

Für eine solche Reaktion kommen auch zweizähnige Liganden als Katalysatorbestandteil zum Einsatz. Dabei kann z.B. der Ligand zusammen mit einem Metall oder in Form eines Komplexes mit einem Metall zum Einsatz kommen.

Unter dem Begriff "zweizähniger Ligand" werden hier und im folgenden Moleküle der allgemeinen Formel

R=P-E-P=R

verstanden, in der
- P=R und R=P, gleich oder verschieden, organische cyclische Gruppen darstellen, in denen die Phosphoratome Teile des cyclischen Systems und über eine Phosphor-Kohlenstoff-Bindung oder eine Phosphor-Sauerstoff-Bindung mit dem cyclischen System verbunden sind; und
- E für eine überbrückende Gruppe steht, die die entsprechenden Phosphoratome beider organischer, cyclischer Gruppen verbindet.

Für die industrielle Umsetzung der Hydroformylierung ist besonders eine hohe Selektivität zu den linearen bzw. verzweigten Aldehyden notwendig. Diese Selektivität wird in der Regel durch das sogenannte l/b-Verhältnis = (linear aldehyde) (branched aldehyde)⁻¹ ausgedrückt. Die Hydroformylierung wird von Frohning und Kohlpaintner in *Applied Homogeneous Catalysis with Organometallic Compounds*, Ed. B. Cornils, W. A. Hermann; VCH, Weinheim **1996**, Vol. 1, S. 29-104 beschrieben.

Ein weiteres Beispiel für die Anwendung von zweizähnigen Liganden in katalytischen Reaktionen ist die Hydrierung, die von Brunner in *Applied Homogeneous Catalysis with Organometallic Compounds*, Ed. B. Cornils, W. A. Hermann; VCH, Weinheim **1996**, Vol. 1, S. 201-219 beschrieben ist.

Die EP-0 530 015 A1 beschreibt die Verwendung von Liganden des Typs R=P-E-P=R, wie z.B. dem Ligand der Formel 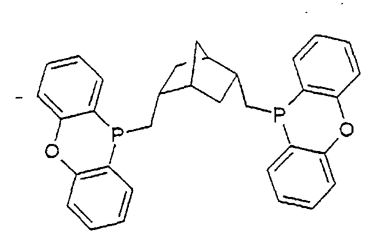 die in Metallkatalysatoren für die chirale Synthese von Pharmazeutika und neuen Intermediaten zum Einsatz kommen.
Die JP 07082281 A2 (JP 93-225998) offenbart, daß Liganden dieses Strukturtyps in der Hydroformylierung zur Synthese von verzweigten Olefinen mit hoher Selektivität eingesetzt werden können.

Hobbs beschreibt in J. Org. Chem. **1981**, Vol. 46, S.4422-4427 den Einsatz eines Liganden des Typs R=P-E-P=R, wie z.B. den Liganden der Formel 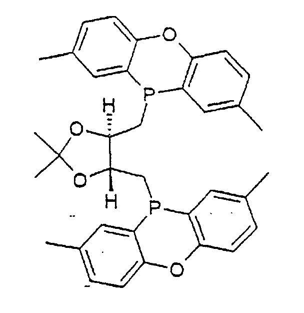 für die asymmetrische Hydroformylierung von Vinylacetat, -propionat und -benzoat, wobei die Selektivität zu den verzweigten Aldehyden 75 - 95 % beträgt.

Die EP-0 213 639 B1 beschreibt einen zweizähnigen Phosphitliganden des Typs R=P-E-P=R, wobei P=R bzw. R=P, gleich oder verschieden, organische cyclische Gruppen darstellen, in denen die Phosphoratome Teile des cyclischen Systems und über eine Phosphor-Sauerstoff-Bindung mit dem cyclischen System verbunden sind, wobei E für eine überbrückende Gruppe steht, die die beiden Phosphoratome der beiden organischen, cyclischen Gruppen verbindet und wobei das Phosphoratom über eine Phosphor-Sauerstoff-Bindung mit der überbrückenden Gruppe E verbunden ist. Diese Liganden, wie z.B. der Ligand der Formel 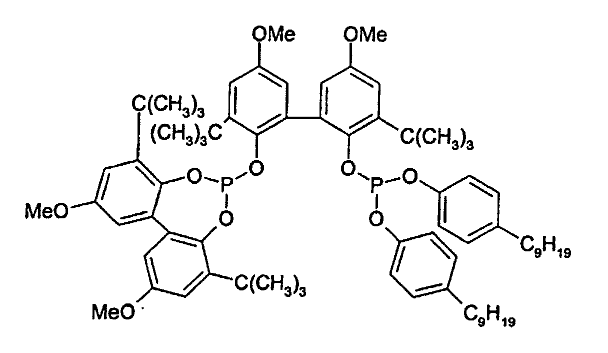 können für die Hydroformylierung von internen Olefinen zu linearen Aldehyden eingesetzt werden.
Die mehrstufige Synthese dieses Liganden und die geringere Stabilität von Phosphitliganden gegenüber Phosphinliganden im allgemeinen ist jedoch für die industrielle Umsetzung ein Nachteil.

Die Aufgabe der vorliegenden Erfindung ist die Bereitstellung von Liganden für die Hydroformylierung von internen Olefinen zu linearen Aldehyden, die die Nachteile der beschriebenen Verfahren zur Hydroformylierung von internen Olefinen überwindet und der interne Olefine zu linearen Aldehyden mit hoher Selektivität umsetzt.

Diese Aufgabe wird gelöst durch einen zweizähnigen Phosphinliganden der allgemeinen Formel (I) 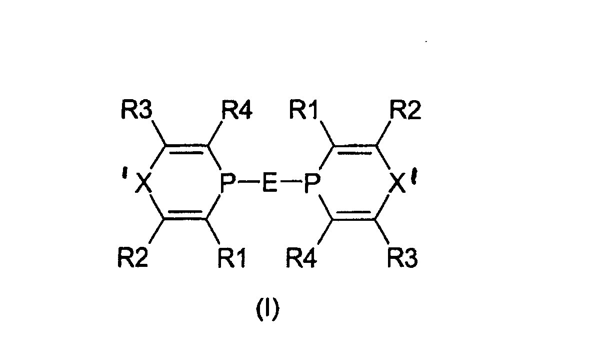 in der:
- die Reste R1, R2, R3 und R4 unabhängig voneinander ein Wasserstoffoder Fluoratom oder einen der folgenden Reste darstellen: (C₁-C₈)-Alkyl, (C₁-C₈)-Alkoxy, (C₁-C₈)-Acyloxy, (C₆-C₁₈)-Aryl, (C₆-C₁₈)-Aryloxy, -CN, -CF₃, -CHO, -SO₃H, -SO₃M, -SO₂R, -SOR, -NH₂, -NH-(C₁-C₈)-Alkyl, -N-(C₁-C₈)-Alkyl₂, -NHCO-(C₁-C₄)-Alkyl, -N-((C₁-C₄)-Alkyl)-(CO-(C₁-C₄)-Alkyl), -COO-(C₁-C₈)-Alkyl, -CONH₂, -CO-(C₁-C₈)-Alkyl, -NHCOH, -NHCOO-(C₁-C₄)-Alkyl, -CO-(C₆-C₁₈)-Aryl, -COO-(C₆-C₁₈)-Aryl, -CHCH-CO₂-(C₁-C₈)-Alkyl, -PO((C₆-C₁₈)-Aryl)₂, -PO-((C₁-C₄)Alkyl)₂; wobei M ein Kation ist, ausgewählt aus der Gruppe der Alkali- oder Erdalkaliionen sowie der Reste NR₂H₂, NR₃H, NRH₃, NR₄, NH₄, PR₂H₂, PR₃H, PRH₃, PR₄ oder/und PH₄;
- die Reste R1, R2, R3 und R4 gegebenenfalls untereinander zusammen einen oder mehrere aliphatische oder aromatische Ringe mit 5 bis 20 Kohlenstoffatomen bilden;
- der Rest E eine der folgenden Gruppen darstellt: 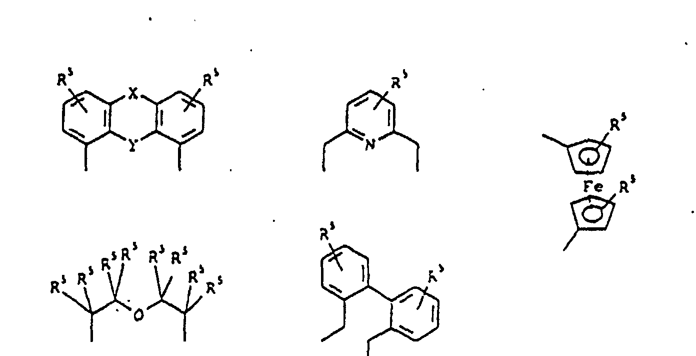 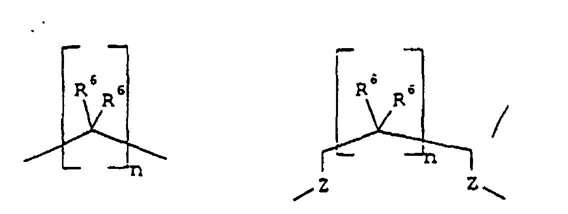 worin
   - X ausgewählt ist aus der Gruppe von -O-, -S-, -Si(R^{a})₂-, Si(OR^{a})₂-, -N(C(O)R^{a})-, -N(R^{b})-, -C(R^{c})(R^{c})-, -C(O)-, -N(SiR^{d})-, -P(R^{d})-, -P(O)(R^{d})-, -C=C(R^{c})(R^{c})- und -P(OR^{d})-, worin
      ■ R^{a} einen (C₁-C₈)-Alkylrest,
      ■ R^{b} einen (C₆-C₁₈)-Arylrest,
      ■ R^{c} ein Wasserstoffatom oder einen (C₁-C₈)-Alkyl-, (C₆-C₁₈)-Arylrest, (C₁-C₈)-Alkoxy-, (C₆-C₁₈)-Aryloxy-, R^{a}(O)- oder R^{b}(O)-Rest darstellt; und
      ■ R^{d} einen der Reste R^{a} oder R^{b} darstellt;
   - Y ein Sauerstoff- oder Schwefelatom darstellt; und
   - die Reste R5, gleich oder verschieden, einen (C₁-C₁₈)-Aryl- oder (C₁-C₁₈)-Alkylrest darstellen;
   - R6 einen (C₁-C₈)-Alkyl- oder (C₆-C₁₈)-Arylrest darstellt;
   - Z ein Sauerstoff- oder Stickstoffatom darstellt; und
   - n eine ganze Zahl zwischen 2 und 6 ist;
   • X¹ ausgewählt ist aus der Gruppe von -O-, -S-, -Si(R^{a})₂-, Si(OR^{a})₂-, -N(C(O)R^{a})-, -N(R^{b})-, -C(R^{c})(R^{c})-, -C(O)-, -N(SiR^{d})-, -P(R^{d})-, -P(O)(R^{d})-, -C=C(R^{c})(R^{c})- und -P(OR^{d})-, worin
   ■ R^{a} einen (C₁-C₈)-Alkylrest,
   ■ R^{b} einen (C₆-C₁₈)-Arylrest,
   ■ R^{c} ein Wasserstoffatom oder einen (C₁-C₈)-Alkyl-, (C₆-C₁₈)-Arylrest, (C₁-C₈)-Alkoxy-, (C₆-C₁₈)-Aryloxy-, R^{a}(O)- oder R^{b}(O)-Rest darstellt; und
   ■ R^{d} einen der Reste R^{a} oder R^{b} darstellt:

Die erfindungsgemäßen Phosphinliganden werden insbesondere eingesetzt bei einem Verfahren zur Herstellung linearer Aldehyde durch Hydroformylierung interner Olefine in Gegenwart eines zweizähnigen Phosphinligand der allgemeinen Formel (II) 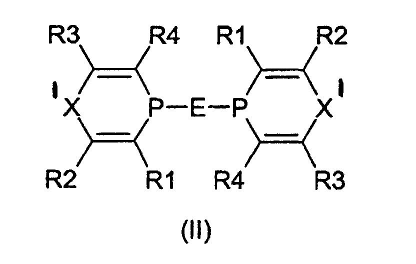 in der:
- die Reste R1, R2, R3 und R4 unabhängig voneinander ein Wasserstoffoder Fluoratom oder einen der folgenden Reste darstellen: (C₁-C₈)-Alkyl, (C₁-C₈)-Alkoxy, (C₁-C₈)-Acyloxy, (C₆-C₁₈)-Aryl, (C₆-C₁₈)-Aryloxy, -CN, -CF₃, -CHO, -SO₃H, -SO₃M, -SO₂R, -SOR, -NH₂, -NH-(C₁-C₈)-Alkyl, -N-(C₁-C₈)-Alkyl₂, -NHCO-(C₁-C₄)-Alkyl, -N-((C₁-C₄)-Alkyl)-(CO-(C₁-C₄)-Alkyl), -COO-(C₁-C₈)-Alkyl, -CONH₂, -CO-(C₁-C₈)-Alkyl, -NHCOH, -NHCOO-(C₁-C₄)-Alkyl, -CO-(C₆-C₁₈)-Aryl, -COO-(C₆-C₁₈)-Aryl, -CHCH-CO₂-(C₁-C₈)-Alkyl, -PO((C₆-C₁₈)-Aryl)₂, -PO-((C₁-C₄)Alkyl)₂; wobei M ein Kation ist, ausgewählt aus der Gruppe der Alkali- oder Erdalkaliionen sowie der Reste NR₂H₂, NR₃H, NRH₃, NR₄, NH₄, PR₂H₂, PR₃H, PRH₃, PR₄ oder/und PH₄;
- die Reste R1, R2, R3 und R4 gegebenenfalls untereinander zusammen einen oder mehrere aliphatische oder aromatische Ringe mit 5 bis 20 Kohlenstoffatomen bilden;
- der Rest E eine der folgenden Gruppen darstellt: 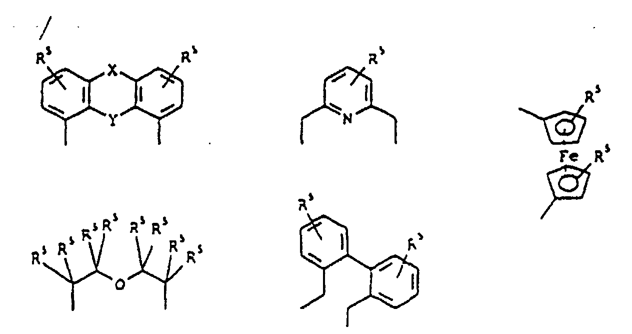 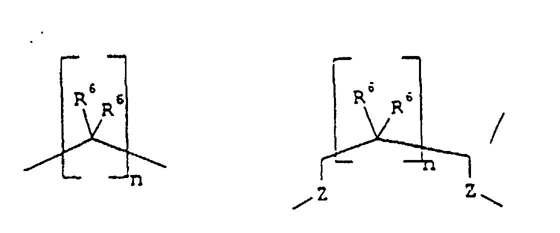 worin:
   - X ausgewählt ist aus der Gruppe von -O-, -S-, -Si(R^{a})₂-, -Si(OR^{a})₂-, -N(C(O)R^{a})-, -N(R^{b})-, -C(R^{c})(R^{c})-, -C(O)-, -N(SiR^{d})-, -P(R^{d})-, -P(O)(R^{d})-, -C=C(R^{c})(R^{c})- und -P(OR^{d})-, worin
      ■ R^{a} einen (C₁-C₈)-Alkylrest,
      ■ R^{b} einen (C₆-C₁₈)-Arylrest,
      ■ R^{c} ein Wasserstoffatom oder einen (C₁-C₈)-Alkyl-, (C₆-C₁₈)-Arylrest, (C₁-C₈)-Alkoxy-, (C₆-C₁₈)-Aryloxy-, R^{a}(O)- oder R^{b}(O)-Rest darstellt; und
      ■ R^{d} einen der Reste R^{a} oder R^{b} darstellt;
   - Y ein Sauerstoff- oder Schwefelatom darstellt; und
   - die Reste R5, gleich oder verschieden, einen (C₁-C₁₈)-Aryl- oder (C₁-C₁₈)-Alkylrest darstellen;
   - R6 einen (C₁-C₈)-Alkyl- oder (C₆-C₁₈)-Arylrest darstellt;
   - Z ein Sauerstoff- oder Stickstoffatom darstellt; und
   - n eine ganze Zahl zwischen 2 und 6 ist;
- X' ausgewählt ist aus der Gruppe von -O-, -S-, -Si(R^{a})₂-, -Si(OR^{a})₂-, -N(C(O)R^{a})-, -N(R^{b})-, -C(R^{c})(R^{c})-, -C(O)-, -N(SiR^{d})-, -P(R^{d})-, -P(O)(R^{d})-, -C=C(R^{c})(R^{c})- und -P(OR^{d})-, worin
   ■ R^{a} einen (C₁-C₈)-Alkylrest,
   ■ R^{b} einen (C₆-C₁₈)-Arylrest,
   ■ R^{c} ein Wasserstoffatom oder einen (C₁-C₈)-Alkyl-, (C₆-C₁₈)-Arylrest, (C₁-C₈)-Alkoxy-, (C₆-C₁₈)-Aryloxy-, R^{a}(O)- oder R^{b}(O)-Rest darstellt; und
   ■ R^{d} einen der Reste R^{a} oder R^{b} darstellt;

Als besonders günstig hat sich erwiesen, wenn die Umsetzung in Gegenwart von Rhodium mit einer Konzentration von 1 bis 1.000 ppm, insbesondere von 10 bis 250 ppm, bezogen auf die gesamte Reaktionsmischung, durchgeführt wird.

Das Verhältnis von Rhodium zu Ligand kann hierbei zwischen 1:1 und 1:100, insbesondere zwischen 1:1 und 1:20, liegt.

Die Temperatur während der Umsetzung liegt im allgemeinen zwischen 10 und 180 °C, insbesondere zwischen 80 und 140 °C, der Druck zwischen 0,1 und 200 bar, insbesondere zwischen 1 und 100 bar.

Die Umsetzung kann in Gegenwart eines Lösungsmittels durchgeführt werden, das insbesondere ausgewählt ist aus der Gruppe von Ether, CO₂, Fluorkohlenwasserstoffen, Toluol und Benzol.
Das Lösungsmittel kann aber auch ein polares, aprotisches Lösungsmittel sein, das insbesondere ausgewählt ist aus der Gruppe von DMAC, DMF oder NMP.
Ebenso ist es möglich, die Umsetzung in Gegenwart eines oligomeren linearen Aldehyds, insbesondere in Gegenwart des Trimeren des herzustellenden linearen Aldehyds, durchzuführen, der hier auch als Lösungsmittel fungiert.

Desweiteren hat es sich als günstig erwiesen, die Umsetzung in einer zweiphasigen Mischung aus dem Lösungsmittel und Wasser durchgeführt wird.

Das CO/H₂-Verhältnis liegt während der erfindungsgemäßen Hydroformylierung üblicherweise zwischen 1:10 und 10:1, insbeondere zwischen 1:2 und 2:1.

Die folgenden Beispiele dienen der Erläuterung der Erfindung:

### Beispiele:

### Allgemeine Darstellungsmethode:

Alle Versuche werden unter Verwendung von Standard-Schlenk-Techniken unter Argonatmosphäre durchgeführt. Die Chemikalien wurden von den Firmen Acros Chimica und Aldrich Chemical Co. bezogen. Rh(CO)₂(dipivaloylmethanoat) wird gemäß den in der Literatur beschriebenen Verfahren hergestellt (H.T. Teunissen, F. Bickelhaupt "*Phosphor, Sulfur*" **1996,** *118*, S. 309-312; H.K.A.C. Coolen, P.W.N.M. van Leeuwen, R.J.M. Nolte "*J. Org. Chem.*" **1996,** *61*, S. 4739-4747). Die NMR-Messungen wurden mit einem Spektrometer der Firma Bruker AMX 300 durchgeführt.

### Herstellung der Diphosphinliganden 2a-2c:

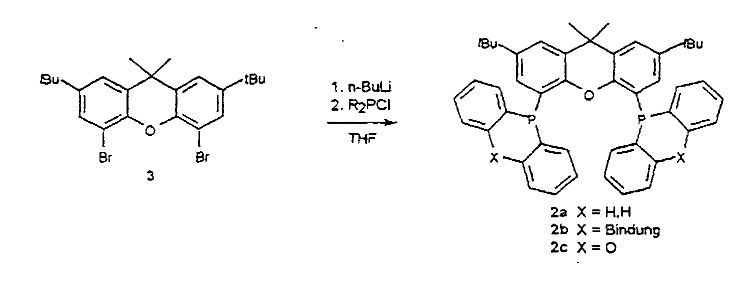

3,7 ml einer Lösung von N-Butyllithium in Hexan (2,5 Mol, 9,3 mMol) wurden zu einer Mischung von 2,00 g 4,5-Dibromo-2,7-di-t-butyl-9,9-dimethylxanthen (Verbindung 3) (4,16 mMol) in 50 ml THF bei -60°C gegeben. Nachdem die erhaltene, weiße Suspension 2 Stunden lang gerührt wurde, wurden 2,2 Äquivalente von Chlorphosphin in 25 ml Toluol hinzugegeben. Die Reaktionsmischung wurde langsam auf Umgebungstemperatur aufgewärmt und über Nacht gerührt. Das Lösemittel wurde unter Vakuum entfernt und der Rest in einer Mischung von Toluol und gesättigter Kochsalzlösung im Verhältnis von 2:1 aufgelöst. Die organische Phase wurde abgetrennt und die wäßrige Phase wurde dreimal mit Toluol extrahiert. Nachdem die vereinigten organischen Extrakte unter Vakuum evakuiert wurden und der so erhaltene Rückstand mit Hexan gewaschen wurde, konnte ein weißes Pulver isoliert werden. Die Liganden wurden in Reinform nach Umkristallisieren erhalten: **2a** aus Ethanol (Ausbeute 64 %), **2b** aus DCM (Ausbeute 52 %) und **2c** aus Toluol (Ausbeute 75 %).
Die physikalischen Parameter der erhaltenen Verbindungen sind im folgenden wiedergegeben:

### Eigenschaften von 2a:

Schmp.: 194 - 195 °C
^{**1**}**H-NMR** (in CDCl₃):
δ [ppm] = 7,38 (d, ⁴*J*(H,H) = 2,2 Hz, 2H; H^{1,8}), 7,24 (m, 20H; Phenyl), 6,53 (bd, ⁴*J*(H,H) = 2,1 Hz, 2H; H^{3,6}), 1,68 (s, 6H; CH₃), 1,11 (s, 18H; t-Butyl).
^{**13**}**C{**^{**1**}**H}-NMR** (in CDCl₃):
δ [ppm]= 150,7 (t, *J*(P,C) = 19,1 Hz; CO), through-space P-P Kopplung = 25,2 Hz, 145,4 (C^{2,7}), 137,0 (t, *J*(P,C) = 13,1 Hz; PC), 134,2 (t, *J*(P,C) = 20,4 Hz; PCCH), 129,7 (C^{3,6}), 129,1 (CC⁹), 128,3 (d, *J*(P,C) = 6,0 Hz; PCCHCH), 128,3 (PCCHCHCH), 124,9 (t, *J*(P,C) = 18,9 Hz; C^{4,5}), 123,2 (C^{1,8}), 35,1 (C⁹), 34,7 (C(CH₃)₃), 32,4 (C⁹CH₃), 31,5 (C(CH₃)₃).
^{**31**}**P{**^{**1**}**H}-NMR** (in CDCl₃):
δ [ppm]=-16,2
Elementaranalyse: C 81,78 % (81,71 %), H 6,69 % (7,01 %)

### Eigenschaften von 2b:

Schmp.: 330 °C
^{**1**}**H-NMR** (in CDCl₃):
δ [ppm] = 8,35 (dd, ³*J*(H,H) = 7,5 Hz, ⁴*J*(H,H) = 1,6 Hz, 4H; DBP-H¹), 7,97 (d, ³*J*(H,H) = 7,5 Hz, 4H; DBP-H⁴), 7,49 (dt, ³*J*(H,H) = 7,5 Hz, ⁴*J*(H,H) = 1,4 Hz, 4H; DBP-H²), 7,41 (dt, ³*J*(H,H) = 7,4 Hz, ⁴*J*(H,H) = 1,3 Hz, 4H; DBP-H³), 7,38 (d, ⁴*J*(H,H) = 2,4 Hz, 2H; H^{1,8}), 6,76 (dt, ⁴*J*(H,H) = 2,3 Hz, *J*(P,H) = 2,5 Hz, 2H; H^{3,6}), through-space P-P Kopplung = 37,8 Hz, 1,69 (s, 6H; CH3), 1,12 (s, 18H; t-Butyl).
^{**13**}**C{**^{**1**}**H}-NMR** (in CDCl₃):
δ [ppm] = 151,0 (t, *J*(P,C) =19,6 Hz; CO), 146 (C^{2,7}), 144,1 (PCC), 142,5 (t, *J*(P,C) = 4,5 Hz; PC), 131,9 (t, *J*(P,C) = 26,4 Hz; DBP-C⁴), 129,5 (CC⁹), 128,7 (DBP-C²), 127,5 (t, *J*(P,C) = 3,0 Hz; DBP-C³), 126,5 (C^{3,6}), 124,5 (C^{1,8}), 124,5 (m, *J*(P,C) = 25,7 Hz; C^{4,5}), 121,6 (DBP-C¹), 35,1 (C⁹), 34,9 (C(CH₃)₃), 33,2 (C⁹CH₃), 31,6 (C(CH₃)₃).
^{**31**}**P{**^{**1**}**H}-NMR** (in CDCl₃):
δ [ppm] = -20,8
Elementaranalyse: C 81,54 % (82,19 %), H 5,73 % (6,46 %)

### Eigenschaften von 2c:

Schmp.: 336 - 338 °C
^{**1**}**H-NMR** (in CDCl₃):
δ [ppm] = 8,18 (t, ³*J*(H,H) = 7,2 Hz, ³*J*(P,H) = 14,4 Hz, 4H; PP-H¹), through-space P-P Kopplung = 65 Hz, 7,40 (dt, ³*J*(H,H) = 7,7 Hz, ⁴*J*(H,H) = 1,3 Hz, 4H; PP-H³), 7,26 (s, 2H; H^{1,8}), 7,24 (d, ³*J*(H,H) = 8,2 Hz, 4H; PP-H⁴), 7,17 (t, ³*J*(H,H) = 7,3 Hz, 4H; PP-H²), 6,67 (s, 2H; H^{3,6}), 1,55 (s, 6H; CH₃), 1,10 (s, 18H; t-Butyl).
^{**13**}**C{**^{**1**}**H}-NMR** (CDCl₃):
δ [ppm] = 155,8 (PP-CO), 149,6 (t, *J*(P,C) = 21,3 Hz; CO), 145,1 (C^{2,7}), 135,5 (t, *J*(P,C) = 43,1 Hz; PP-C¹), 130,5 (PP-C³), 128,8 (CC⁹), 128,5 (C^{3,6}), 125,7 (t, *J*(P,C) = 29,0 Hz; PP-PC), 123,8 (C^{1,8}), 123,3 (t, *J*(P,C) = 11,1 Hz; PP-C²), 118,3 (C^{4,5}), 117,4 (PP-C⁴), 34,4 (C⁹), 34,1 (C(CH₃)₃), 32,7 (C⁹CH₃), 30,9 (C(CH₃)₃).
^{**31**}**P{**^{**1**}**H}-NMR** (CDCl₃):
δ [ppm] = -69.9
Elementaranalyse: C 78,47 % (78,53 %). H 5,6 % (6,17 %)

Die zuvor beschriebenen Diphosphinliganden **2a, 2b** und **2c** wurden gemäß den nachfolgend beschriebenen Bedingung bei der Hydroformylierung interner Olefine verwendet:

**Tabelle 1**

| Ergebnisse der Hydroformylierung von 1-Octen: | | | | | | |
|---|---|---|---|---|---|---|
| Ligand | t (min) | Umsetzung (%) | Selektivität (Isomere in %) | l/b | Selektivität (lin. Aldehyd in %) | TOF |
| PPh3 | 5,3 | 26 | 1,2 | 3,1 | 74 | 1880 |
| **2a** | 30 | 21 | 3,9 | 49 | 94 | 250 |
| **2b** | 24 | 28 | 16 | 65 | 83 | 360 |
| **2c** | 8,5 | 27 | 13 | 68 | 86 | 1100 |

Die Reaktionen wurden in einem 180 ml Edelstahlautoklaven in Toluol bei 80 °C unter einer CO/H₂-Atmosphäre (Verhältnis 1:1) mit einem anfänglichen Druck von 20 bar durchgeführt, der Katalysatorprecursor ist Rh(CO)₂(dipivaloylmethanoat), die Rhodiumkonzentration beträgt 1,0 mMol, das Verhältnis von Rh:P:1-Octen beträgt 1:10:673.

Die Umsetzung, das l/b-Verhältnis und die Selektivitäten zu den isomerisierten Olefinen und zu dem linearen Aldehyd werden durch Gaschromatographie unter

Verwendung von Dekan als interner Standard bestimmt.

TOF bedeutet hier die Turnoverfrequenz, die berechnet wird als (Molaldehyd)(Molkatalysator)⁻¹(h)⁻¹.

**Tabelle 2.**

| Ergebnisse der Hydroformylierung von *trans* 2- und 4-Octen: | | | | | | |
|---|---|---|---|---|---|---|
| Ligand | Substrat | t (h) | Umsetzung (%) | l/b | Selektivität (lin. Aldehyd in %) | TOF |
| **2b** | 2-Octen | 1,0 | 10 | 9,5 | 90 | 65 |
| **2c** | 2-Octen | 1,0 | 22 | 9,2 | 90 | 112 |
| **2b** | 4-Octen | 17 | 54 | 6,1 | 86 | 15 |
| **2c** | 4-Octen | 17 | 67 | 4,4 | 81 | 20 |

Die Reaktionsbedingungen sind identisch mit denen von Tabelle 1 mit der Ausnahme, daß die Temperatur 120 °C und der Druck 2 bar beträgt.

**Tabelle 3.**

| Ergebnisse der Hydroformylierung von n-Oktengemisch: | | | | | |
|---|---|---|---|---|---|
| Ligand | t (h) | Umsetzung (%) | l/b | Selektivität (lin. Aldehyd in %) | TOF |
| PPh₃ | 2,8 | 57 | 0,5 | 33 | 318 |
| **2c** | 2,8 | 63 | 1,8 | 64 | 393 |
| **2c**^{**a**} | 2,0 | 66 | 1,4 | 58 | 517 |
| **2c** | 1,7 | 68 | 1,7 | 63 | 648 |

| | | | | | |
|---|---|---|---|---|---|
| ^{a} Druck 20 bar. | | | | | |

Die Reaktionsbedingungen sind identisch mit denen von Tabelle 2 mit der

Ausnahme, daß der Druck 10 bar beträgt.

**Tabelle 4**

| Ergebnisse der Hydroformylierung von Butengemisch: | | | | | |
|---|---|---|---|---|---|
| Ligand | t (h) | Umsetzung (%) | l/b | Selektivität (lin. Aldehyd in %) | TOF |
| **2c** | 1,5 | 82 | 6,7 | 87 | 985 |
| **2c**^{**a**} | 1,5 | 84 | 4,6 | 82 | 1050 |

| | | | | | |
|---|---|---|---|---|---|
| ^{a} Druck 20 bar. | | | | | |

Die Reaktionsbedingungen sind identisch mit denen von Tabelle 2 mit der Ausnahme, daß der Druck 10 bar beträgt.

## Patentansprüche

1. Zweizähniger Phosphinligand der allgemeinen Formel (I) 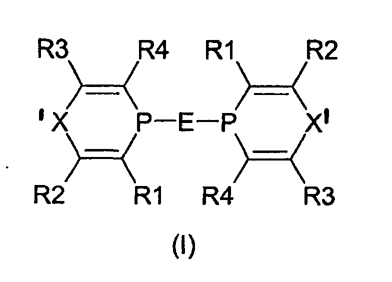 in der:
• die Reste R1, R2, R3 und R4 unabhängig voneinander ein Wasserstoff- oder Fluoratom oder einen der folgenden Reste darstellen: (C₁-C₈)-Alkyl, (C₁-C₈)-Alkoxy, (C₁-C₈)-Acyloxy, (C₆-C₁₈)-Aryl, (C₆-C₁₈)-Aryloxy, -CN, -CF₃, -CHO, -SO₃H, -SO₃M, -SO₂R,-SOR, -NH₂, -NH-(C₁-C₈)-Alkyl, -N-(C₁-C₈)-Alkyl₂, -NHCO-(C₁-C₄)-Alkyl, -N-((C₁-C₄)-Alkyl)-(CO-(C₁-C₄)-Alkyl), -COO-(C₁-C₈)-Alkyl, -CONH₂, -CO-(C₁-C₈)-Alkyl, -NHCOH, -NHCOO-(C₁-C₄)-Alkyl, -CO-(C₆-C₁₈)-Aryl, -COO-(C₆-C₁₈)-Aryl, -CHCH-CO₂-(C₁-C₈)-Alkyl, -PO((C₆-C₁₈)-Aryl)₂, -PO-((C₁-C₄)Alkyl)₂; wobei M ein Kation ist, ausgewählt aus der Gruppe der Alkali- oder Erdalkaliionen sowie der Reste NR₂H₂, NR₃H, NRH₃, NR₄, NH₄, PR₂H₂, PR₃H, PRH₃, PR₄. oder/und PH₄;
• die Reste R1, R2, R3 und R4 gegebenenfalls untereinander einen oder mehrere aliphatische oder aromatische Ringe mit 5 bis 20 Kohlenstoffatomen bilden;
• der Rest E eine der folgenden Gruppen darstellt: 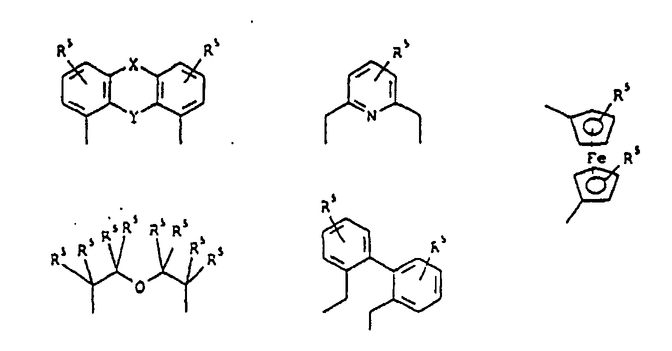 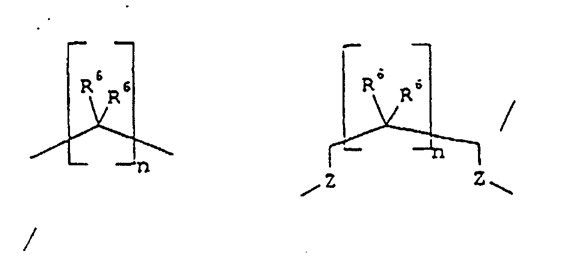 worin:
• X ausgewählt ist aus der Gruppe von -O-, -S-, -Si(R^{a})₂-, Si(OR^{a})₂-, -N(C(O)R^{a})-, -N(R^{b})-, -C(R^{c})(R^{c})-, -C(O)-, -N(SiR^{d})-, -P(R^{d})-, -P(O)(R^{d})-, -C=C(R^{c})(R^{c})- und -P(OR^{d})-, worin
■ R^{a} einen (C₁-C₈)-Alkylrest,
■ R^{b} einen (C₆-C₁₈)-Arylrest,
■ R^{c} ein Wasserstoffatom oder einen (C₁-C₈)-Alkyl-, (C₆-C₁₈)-Arylrest, (C₁-C₈)-Alkoxy-, (C₆-C₁₈)-Aryloxy-, R^{a}(O)- oder R^{b}(O)-Rest darstellt; und
■ R^{d} einen der Reste R^{a} oder R^{b} darstellt;
• Y ein Sauerstoff- oder Schwefelatom darstellt; und
• die Reste R5, gleich oder verschieden, einen (C₁-C₁₈)-Aryl- oder (C₁-C₁₈)-Alkylrest darstellen;
• R6 einen (C₁-C₈)-Alkyl- oder (C₆-C₁₈)-Arylrest darstellt;
• Z ein Sauerstoff- oder Stickstoffatom darstellt; und
• n eine ganze Zahl zwischen 2 und 6 ist;
• ¹X ausgewählt ist aus der Gruppe von -O-, -S-, -Si(R^{a})₂-, -Si(OR^{a})₂-, -N(C(O)R^{a})-, -N(R^{b})-, -C(R^{c})(R^{c})-, -C(O)-, -N(SiR^{d})-, -P(R^{d})-, -P(O)(R^{d})-, -C=C(R^{c})(R^{c})- und -P(OR^{d})-, worin
■ R^{a} einen (C₁-C₈)-Alkylrest,
■ R^{b} einen (C₆-C₁₈)-Arylrest,
■ R^{c} ein Wasserstoffatom oder einen (C₁-C₈)-Alkyl-, (C₆-C₁₈)-Arylrest, (C₁-C₈)-Alkoxy-, (C₆-C₁₈)-Aryloxy-, R^{a}(O)- oder R^{b}(O)-Rest darstellt; und
■ R^{d} einen der Reste R^{a} oder R^{b} darstellt.

2. Verfahren zur Herstellung linearer Aldehyde durch Hydroformylierung interner Olefine in Gegenwart eines zweizähnigen Phosphinligand der allgemeinen Formel (II) 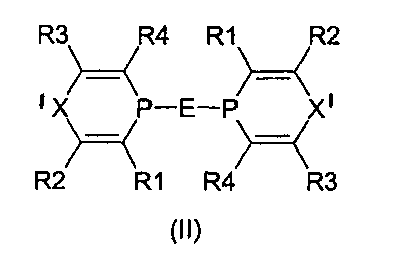 in der:
• die Reste R1, R2, R3 und R4 unabhängig voneinander ein Wasserstoff- oder Fluoratom oder einen der folgenden Reste darstellen: (C₁-C₈)-Alkyl, (C₁-C₈)-Alkoxy, (C₁-C₈)-Acyloxy, (C₆-C₁₈)-Aryl, (C₆-C₁₈)-Aryloxy, -CN, -CF₃, -CHO, -SO₃H, -SO₃M, -SO₂R, -SOR, -NH₂, -NH-(C₁-C₈)-Alkyl, -N-(C₁-C₈)-Alkyl₂, -NHCO-(C₁-C₄)-Alkyl, -N-((C₁-C₄)-Alkyl)-(CO-(C₁-C₄)-Alkyl), -COO-(C₁-C₈)-Alkyl, -CONH₂, -CO-(C₁-C₈)-Alkyl, -NHCOH, -NHCOO-(C₁-C₄)-Alkyl, -CO-(C₆-C₁₈)-Aryl, -COO-(C₆-C₁₈)-Aryl, -CHCH-CO₂-(C₁-C₈)-Alkyl, -PO((C₆-C₁₈)-Aryl)₂, -PO-((C₁-C₄)Alkyl)₂; wobei M ein Kation ist, ausgewählt aus der Gruppe der Alkali- oder Erdalkaliionen sowie der Reste NR₂H₂, NR₃H, NRH₃, NR₄, NH₄, PR₂H₂, PR₃H, PRH₃, PR₄ oder/und PH₄;
• die Reste R1, R2, R3 und R4 gegebenenfalls untereinander zusammen einen oder mehrere aliphatische oder aromatische Ringe mit 5 bis 20 Kohlenstoffatomen bilden;
• der Rest E eine der folgenden Gruppen darstellt: 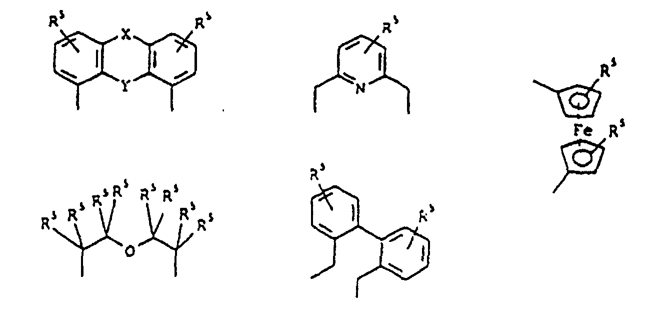 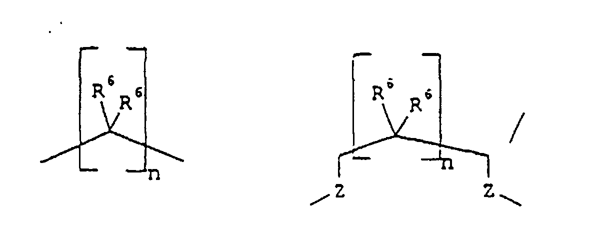 worin:
• X ausgewählt ist aus der Gruppe von -O-, -S-, -Si(R^{a})₂-, -Si(OR^{a})₂-, -N(C(O)R^{a})-, -N(R^{b})-, -C(R^{c})(R^{c})-, -C(O)-, -N(SiR^{d})-, -P(R^{d})-, -P(O)(R^{d})-, -C=C(R^{c})(R^{c})- und -P(OR^{d})-, worin
■ R^{a} einen (C₁-C₈)-Alkylrest,
■ R^{b} einen (C₆-C₁₈)-Arylrest,
■ R^{c} ein Wasserstoffatom oder einen (C₁-C₈)-Alkyl-, (C₆-C₁₈)-Arylrest, (C₁-C₈)-Alkoxy-, (C₆-C₁₈)-Aryloxy-, R^{a}(O)- oder R^{b}(O)-Rest darstellt; und
■ R^{d} einen der Reste R^{a} oder R^{b} darstellt;
• Y ein Sauerstoff- oder Schwefelatom darstellt; und
• die Reste R5, gleich oder verschieden, einen (C₁-C₁₈)-Aryl- oder (C₁-C₁₈)-Alkylrest darstellen;
• R6 einen (C₁-C₈)-Alkyl- oder (C₆-C₁₈)-Arylrest darstellt;
• Z ein Sauerstoff- oder Stickstoffatom darstellt; und
• n eine ganze Zahl zwischen 2 und 6 ist;
• X¹ ausgewählt ist aus der Gruppe von -O-, -S-, -Si(R^{a})₂-, -Si(OR^{a})₂-, -N(C(O)R^{a})-, -N(R^{b})-, -C(R^{c})(R^{c})-, -C(O)-, -N(SiR^{d})-, -P(R^{d})-, -P(O)(R^{d})-, -C=C(R^{c})(R^{c})- und -P(OR^{d})-, worin
■ R^{a} einen (C₁-C₈)-Alkylrest,
■ Rb einen (C₆-C₁₈)-Arylrest,
■ R^{c} ein Wasserstoffatom oder einen (C₁-C₈)-Alkyl-, (C₆-C₁₈)-Arylrest, (C₁-C₈)-Alkoxy-, (C₆-C₁₈)-Aryloxy-, R^{a}(O)- oder R^{b}(O)-Rest darstellt; und
■ R^{d} einen der Reste R^{a} oder R^{b} darstellt.

3. Verfahren nach Anspruch 2, **dadurch gekennzeichnet, daß** die Umsetzung in Gegenwart von Rhodium mit einer Konzentration von 1 bis 1.000 ppm, insbesondere von 10 bis 250 ppm, bezogen auf die gesamte Reaktionsmischung, durchgeführt wird.

4. Verfahren nach Anspruch 3, **dadurch gekennzeichnet, daß** das Verhältnis von Rhodium zu Ligand zwischen 1:1 und 1:100, insbesondere zwischen 1:1 und 1:20, liegt.

5. Verfahren nach einem der Ansprüche 2 bis 4, **dadurch gekennzeichnet, daß** die Umsetzung bei einer Temperatur von 10 bis 180°C, insbesondere von 80 bis 140°C, durchgeführt wird.

6. Verfahren nach einem der Ansprüche 2 bis 5, **dadurch gekennzeichnet, daß** die Umsetzung bei einem Druck von 0,1 bis 200 bar, insbesondere von 1 bis 100 bar, durchgeführt wird.

7. Verfahren nach einem der Ansprüche 2 bis 6, **dadurch gekennzeichnet, daß** die Umsetzung in Gegenwart eines Lösungsmittels durchgeführt wird, das insbesondere ausgewählt ist aus der Gruppe von Ether, CO₂, Fluorkohlenwasserstoffen, Toluol und Benzol.

8. Verfahren nach einem der Ansprüche 2 bis 7, **dadurch gekennzeichnet, daß** die Umsetzung in Gegenwart eines polaren, aprotischen Lösungsmittels durchgeführt wird, das insbesondere ausgewählt ist aus der Gruppe von DMAC, DMF oder NMP.

9. Verfahren nach einem der Ansprüche 2 bis 8, **dadurch gekennzeichnet, daß** die Umsetzung in Gegenwart eines oligomeren linearen Aldehyds, insbesondere in Gegenwart des Trimeren des herzustellenden linearen Aldehyds, durchgeführt wird.

10. Verfahren nach einem der Ansprüche 7 bis 9, **dadurch gekennzeichnet, daß** die Umsetzung in einer zweiphasigen Mischung aus dem Lösungsmittel und Wasser durchgeführt wird.

11. Verfahren nach einem der Ansprüche 2 bis 10, **dadurch gekennzeichnet, daß** das CO/H₂-Verhältnis während der Hydroformylierung zwischen 1:10 und 10:1, insbesondere zwischen 1:2 und 2:1, liegt.

## Claims

1. A bidentate phosphine ligand of the formula (I) 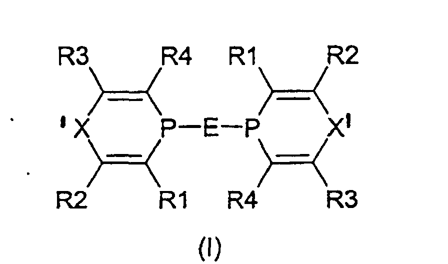 where.
• the radicals R1, R2, R3 and R4 independently of one another are a hydrogen or fluorine atom or one of the following radicals: (C₁-C₈)-alkyl, (C₁-C₈)-alkoxy, (C₁-C₈)-acyloxy, (C₆-C₁₈)-aryl, (C₆-C₁₈)-aryloxy, -CN, -CF₃, -CHO, -SO₃H, -SO₃M, -SO₂R, -SOR, -NH₂, -NH-(C₁-C₈)-alkyl, -N-(C₁-C₈)-alkyl₂, -NHCO-(C₁-C₄)-alkyl, -N-((C₁-C₄-alkyl)-(CO-(C₁-C₄)-alkyl), -COO-(C₁-C₈)-alkyl, -CONH₂, -CO-(C₁-C₈)-alkyl, -NHCOH, -NHCOO-(C₁-C₄)-alkyl, -CO-(C₆-C₁₈)-aryl, -COO-(C₆-C₁₈)-aryl, -CHCH-CO₂-(C₁-C₈)-alkyl, -PO((C₆-C₁₈)-aryl)₂, -PO-((C₁-C₄)-alkyl)₂; where M is a cation selected from the group consisting of the alkali metal ions or alkaline earth metal ions and of the radicals NR₂H₂, NR₃H, NRH₃, NR₄, NH₄, PR₂H₂, PR₃H, PRH₃, PR₄ or/and PH₄;
• the radicals R1, R2, R3 and R4, if appropriate with one another, form one or more aliphatic or aromatic rings having from 5 to 20 carbon atoms;
• the radical E is one of the following groups: 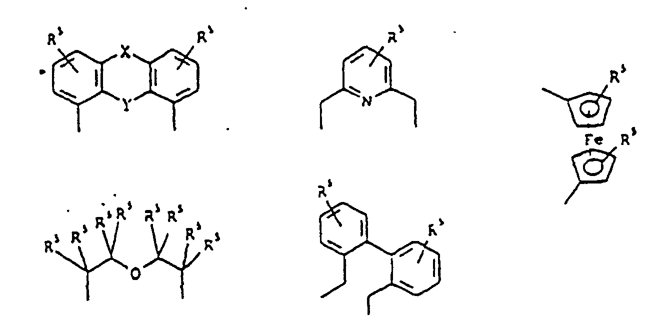 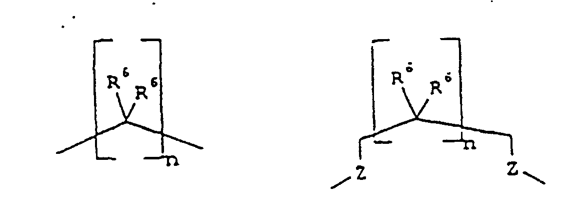 where:
• X is selected from the group consisting of -O-, -S-, -Si(R^{a})₂-, -Si(OR^{a})₂-, -N(C(O)R^{a})-, -N(R^{b})-, -C(R^{c})(R^{c})-, -C(O)-, -N(SiR^{d})-, -P(R^{d})-, -P(O)(R^{d})-, -C=C(R^{c})(R^{c})- and -P(OR^{d})-, where
• R^{a} is a (C₁-C₈)-alkyl radical,
• R^{b} is a (C₆-C₁₈)-aryl radical,
• R^{c} is a hydrogen atom or a (C₁-C₈)-alkyl radical, (C₆-C₁₈)-aryl radical, (C₁-C₈)-alkoxy radical, (C₆-C₁₈)-aryloxy radical,
R^{a}(O)- or R^{b}(O)-radical; and
• R^{d} is one of the radicals R^{a} or R^{b};
• Y is an oxygen or sulfur atom; and
• the radicals R5 are identical or different and are a (C₁-C₁₈)-aryl or (C₁-C₁₈)-alkyl radical;
• R6 is a (C₁-C₈)-alkyl or (C₆-C₁₈)-aryl radical;
• Z is an oxygen or nitrogen atom; and
• n is an integer between 2 and 6;
• ¹X is selected from the group consisting of -O-, -S-, -Si(R^{a})₂-, -Si(OR^{a})₂-, -N(C(O)R^{a})-, -N(R^{b})-, -C(R^{c})(R^{c})-, -C(O)-, -N(SiR^{d})-, -P(R^{d})-, -P(O)(R^{d})-, -C=C(R^{c})(R^{c})- and -P(OR^{d})-, where
• R^{a} is a (C₁-C₈)-alkyl radical,
• R^{b} is a (C₆-C₁₈)-aryl radical,
• R^{c} is a hydrogen atom or a (C₁-C₈)-alkyl radical, (C₆-C₁₈)-aryl radical, (C₁-C₈)-alkoxy radical, (C₆-C₁₈)-aryloxy radical, R^{a}(O)- or R^{b}(O)-radical; and
• R^{d} is one of the radicals R^{a} or R^{b}.

2. A process for preparing linear aldehydes by hydroformylating internal olefins in the presence of a bidentate phosphine ligand of the formula (II) 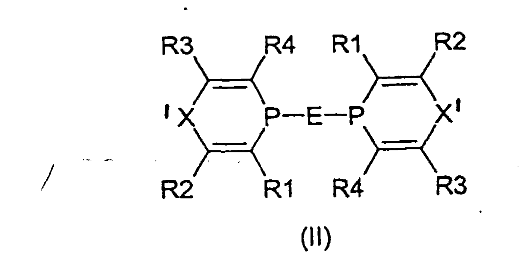 where:
• the radicals R1, R2, R3 and R4 independently of one another are a hydrogen or fluorine atom or one of the following radicals: (C₁-C₈)-alkyl, (C₁-C₈)-alkoxy, (C₁-C₈)-acyloxy, (C₆-C₁₈)-aryl, (C₆-C₁₈)-aryloxy, -CN, -CF₃, -CHO, -SO₃H, -SO₃M, -SO₂R, -SOR, -NH₂, -NH-(C₁-C₈)-alkyl, -N-(C₁-C₈)-alkyl₂, -NHCO-(C₁-C₄)-alkyl, -N-((C₁-C₄)-alkyl)-(CO-(C₁-C₄)-alkyl), -COO-(C₁-C₈)-alkyl, -CONH₂, -CO-(C₁-C₈)-alkyl, -NHCOH, -NHCOO-(C₁-C₄)-alkyl, -CO-(C₆-C₁₈)-aryl, -COO-(C₆-C₁₈)-aryl, -CHCH-CO₂-(C₁-C₈)-alkyl, -PO((C₆-C₁₈)-aryl₂, -PO-((C₁-C4)-alkyl)₂; where M is a cation selected from the group consisting of the alkali metal ions or alkaline earth metal ions and of the radicals NR₂H₂, NR₃H, NRH₃, NR₄, NH₄, PR₂H₂, PR₃H, PRH₃, PR₄ or/and PH₄;
• the radicals R1, R2, R3 and R4, if appropriate with one another, together form one or more alipahtic or aromatic rings having from 5 to 20 carbon atoms;
• the radical E is one of the following groups: 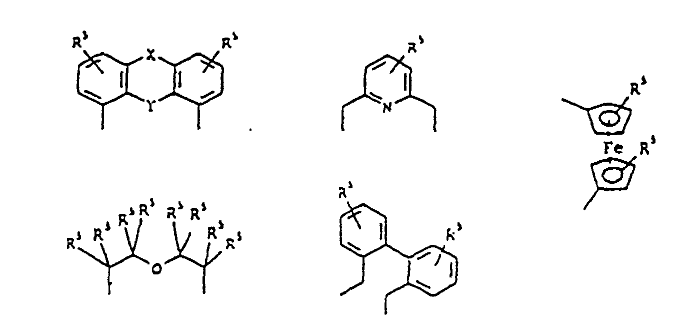 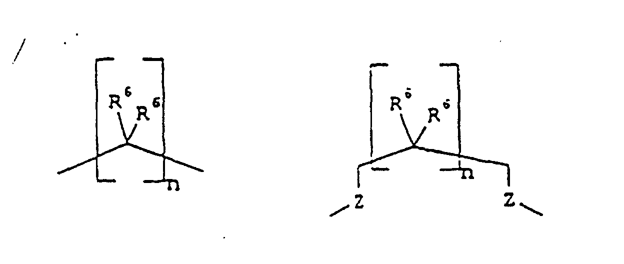 where:
• X is selected from the group consisting of -O-, -S-, -Si(R^{a})₂-, -Si(OR^{a})₂-, -N(C(O)R^{a})-, -N(R^{b})-, C(R^{c})(R^{c})-, -C(O)-, -N(SiR^{d})-, -P(R^{d})-, -P(O)(R^{d})-, -C=C(R^{c})(R^{c})- and -P(OR^{d})-, where
• R^{a} is a (C₁-C₈)-alkyl radical,
• R^{b} is a (C₆-C₁₈)-aryl radical,
• R^{c} is a hydrogen atom or a (C₁-C₈)-alkyl radical, (C₆-C₁₈)-aryl radical, (C₁-C₈)-alkoxy radical, (C₆-C₁₈)-aryloxy radical, R^{a}(O)- or R^{b}(O)-radical; and
• R^{d} is one of the radicals R^{a} or R^{b};
• Y is an oxygen or sulfur atom; and
• the radicals R5 are identical or different and are a (C₁-C₁₈)-aryl or (C₁-C₁₈)-alkyl radical;
• R6 is a (C₁-C₈)-alkyl or (C₆-C₁₈)-aryl radical;
• Z is an oxygen or nitrogen atom; and
• n is an integer between 2 and 6;
• X¹ is selected from the group consisting of -O-, -S-, -Si(R^{a})₂-, -Si(OR^{a})₂-, -N(C(O)R^{a})-, -N(R^{b})-, -C(R^{c})(R^{c})-, -C(O)-, -N(SiR^{d})-, -P(R^{d})-, -P(O)(R^{d})-, C=C(R^{c})(R^{c})- and -P(OR^{d})-, where
• R^{a} is a (C₁-C₈)-alkyl radical,
• R^{b} is a (C₆-C₁₈)-aryl radical,
• R^{c} is a hydrogen atom or a (C₁-C₈)-alkyl radical, (C₆-C₁₈)-aryl radical, (C₁-C₈)-alkoxy radical, (C₆-C₁₈)-aryloxy radical, R^{a}(O)- or R^{b}(O)-radical; and
• R^{d} is one of the radicals R^{a} or R^{b}.

3. The process as claimed in claim 2, wherein the reaction is carried out in the presence of rhodium at a concentration of from 1 to 1000 ppm, in particular from 10 to 250 ppm, based on the total reaction mixture.

4. The process as claimed in claim 3, wherein the ratio of rhodium to ligand is between 1:1 and 1:100, in particular between 1:1 and 1:20.

5. The process as claimed in one of claims 2 to 4, wherein the reaction is carried out at a temperature of from 10 to 180°C, in particular from 80 to 140°C.

6. The process as claimed in one of claims 2 to 5, wherein the reaction is carried out at a pressure of from 0.1 to 200 bar, in particular from 1 to 100 bar.

7. The process as claimed in one of claims 2 to 6, wherein the reaction is carried out in the presence of a solvent which is selected, in particular, from the group consisting of ether, CO₂, fluorinated hydrocarbons, toluene and benzene.

8. The process as claimed in one of claims 2 to 7, wherein the reaction is carried out in the presence of a polar aprotic solvent which is selected, in particular, from the group consisting of DMAC, DMF or NMP.

9. The process as claimed in one of claims 2 to 8, wherein the reaction is carried out in the presence of an oligomeric linear aldehyde, in particular in the presence of the trimer of the linear aldehyde to be prepared.

10. The process as claimed in one of claims 7 to 9, wherein the reaction is carried out in a two-phase mixture of the solvent and water.

11. The process as claimed in one of claims 2 to 10, wherein the CO/H₂ ratio during the hydroformylation is between 1:10 and 10:1, in particular between 1:2 and 2:1.

## Revendications

1. Ligand phosphinique bidenté de formule générale (I) 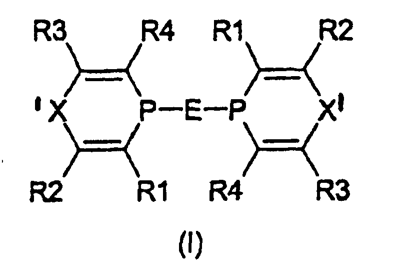 dans laquelle
• les radicaux R1, R2, R3 et R4 représentent, indépendamment les uns des autres, un atome d'hydrogène ou de fluor ou l'un des radicaux suivants : alkyle en C₁-C₈, alcoxy en C₁-C₈, acyloxy en C₁-C₈, aryle en C₆-C₁₈, aryloxy en C₆-C₁₈, -CN, -CF₃, -CHO, -SO₃H, -SO₃M, -SO₂R, -SOR, -NH₂, -NH-alkyle(C₁-C₈), -N-[alkyle(C₁-C₈)]₂, -NHCO-alkyle(C₁-C₄), -N-[alkyle(C₁-C₄)]-[CO-alkyle(C₁-C₄)], -COO-alkyle(C₁-C₈), -CONH₂, -CO-alkyle(C₁-C₈), -NHCOH, -NHCOO-alkyle(C₁-C₄), -CO-aryle(C₆-C₁₈), -COO-aryle(C₆-C₁₈), -CHCH-CO₂-alkyle(C₁-C₈), -PO[aryle(C₆-C₁₈)]₂, -PO-[alkyle(C₁-C₄)]₂ ; M étant un cation choisi dans le groupe des ions alcalins ou alcalino-terreux ainsi que des radicaux NR₂H₂, NR₃H, NRH₃, NR₄, NH₄, PR₂H₂, PR₃H, PRH₃, PR₄ et/ou PH₄ ;
• les radicaux R1, R2, R3 et R4 forment éventuellement ensemble un ou plusieurs cycles aliphatiques ou aromatiques ayant de 5 à 20 atomes de carbone ;
• le radical E représente l'un des groupes suivants : 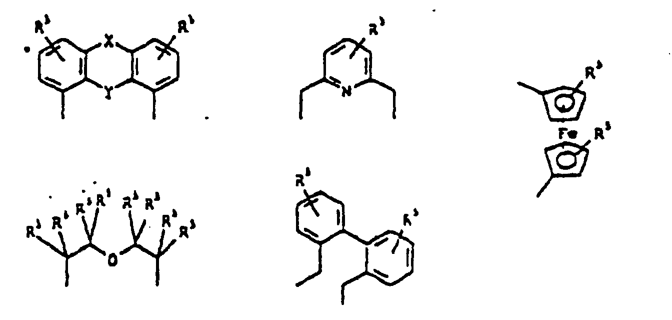 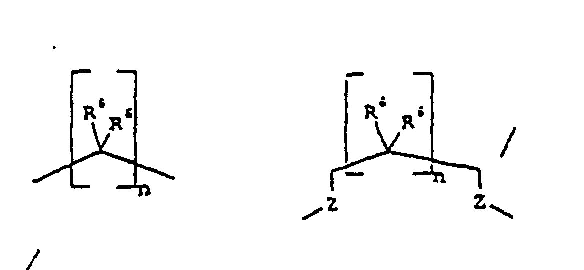 dans lesquels
• X est choisi dans le groupe constitué par -O-, -S-, -Si(R^{a})₂-, -Si(OR^{a})₂-, -N(C(O)R^{a})-, -N(R^{b})-, -C(R^{c})(R^{c})-, -C(O)-, -N(SiR^{d})-, -P(R^{d})-, -P(O)(R^{d})-, -C=C(R^{c})(R^{c})- et -P(OR^{d})-, dans lesquels
■ R^{a} représente un radical alkyle en C₁-C₈,
■ R^{b} représente un radical aryle en C₆-C₁₈,
■ R^{c} représente un atome d'hydrogène ou un radical alkyle en C₁-C₈, aryle en C₆-C₁₈, alcoxy en C₁-C₈, aryloxy en C₆-C₁₈, R^{a}(O)- ou R^{b}(O)- ; et
■ R^{d} représente l'un des radicaux R^{a} et R^{b} ;
• Y représente un atome d'oxygène ou de soufre ; et
• les radicaux R5, identiques ou différents, représentent un radical aryle en C₁-C₁₈ ou alkyle en C₁-C₁₈ ;
• R6 représente un radical alkyle en C₁-C₈ ou aryle en C₆-C₁₈;
• Z représente un atome d'oxygène ou d'azote ; et
• n est un nombre entier compris entre 2 et 6 ;
• X¹ est choisi dans le groupe constitué par -O-, -S-, -Si(R^{a})₂-, -Si(OR^{a})₂-, -N(C(O)R^{a})-, -N(R^{b})-, -C(R^{c})(R^{c})-, -C(O)-, -N(SiR^{d})-, -P(R^{d})-, -P(O)(R^{d})-, -C=C(R^{c})(R^{c})- et -P(OR^{d})-, dans lesquels
■ R^{a} représente un radical alkyle en C₁-C₈,
■ R^{b} représente un radical aryle en C₆-C₁₈,
■ R^{c} représente un atome d'hydrogène ou un radical alkyle en C₁-C₈, aryle en C₆-C₁₈, alcoxy en C₁-C₈, aryloxy en C₆-C₁₈, R^{a}(O)- ou R^{b}(O)- ; et
■ R^{d} représente l'un des radicaux R^{a} et R^{b}.

2. Procédé pour la préparation d'aldéhydes linéaires par hydroformylation d'oléfines internes, en présence d'un ligand phosphinique bidenté de formule générale (II) 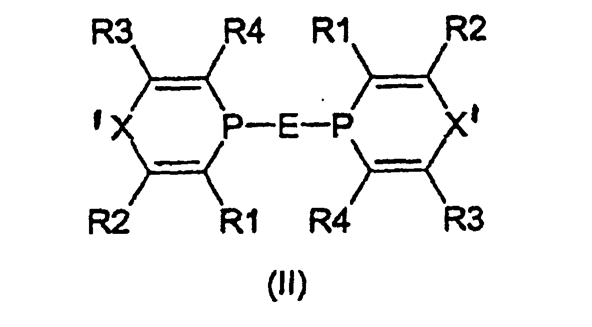 dans laquelle
• les radicaux R1, R2, R3 et R4 représentent, indépendamment les uns des autres, un atome d'hydrogène ou de fluor ou l'un des radicaux suivants : alkyle en C₁-C₈, alcoxy en C₁-C₈, acyloxy en C₁-C₈, aryle en C₆-C₁₈, aryloxy en C₆-C₁₈, -CN, -CF₃, -CHO, -SO₃H, -SO₃M, -SO₂R, -SOR, -NH₂, -NH-alkyle(C₁-C₈), -N-[alkyle(C₁-C₈)]₂, -NHCO-alkyle(C₁-C₄), -N-[alkyle(C₁-C₄)]-[CO-alkyle(C₁-C₄)], -COO-alkyle(C₁-C₈), -CONH₂, -CO-alkyle(C₁-C₈), -NHCOH, -NHCOO-alkyle(C₁-C₄), -CO-aryle(C₆-C₁₈), -COO-aryle(C₆-C₁₈), -CHCH-CO₂-alkyle(C₁-C₈), -PO-[aryle(C₆-C₁₈)]₂, -PO-[alkyle(C₁-C₄)]₂ ; M étant un cation choisi dans le groupe des ions alcalins ou alcalino-terreux ainsi que des radicaux NR2H2, NR₃H, NRH₃, NR4, NH₄, PR₂H₂, PR₃H, PRH₃, PR₄ et/ou PH₄ ;
• les radicaux R1, R2, R3 et R4 forment éventuellement ensemble un ou plusieurs cycles aliphatiques ou aromatiques ayant de 5 à 20 atomes de carbone ;
• le radical E représente l'un des groupes suivants : 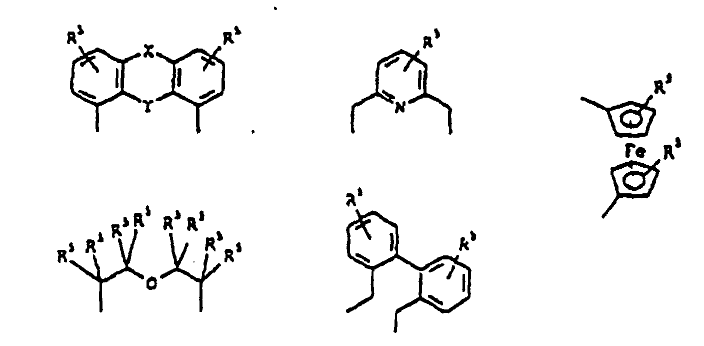 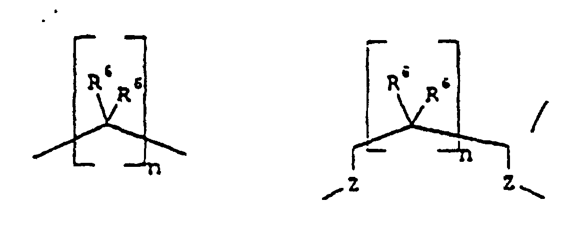 dans lesquels
• X est choisi dans le groupe constitué par -O-, -S-, -Si(R^{a})₂-, -Si(OR^{a})₂-, -N(C(O)R^{a})-, -N(R^{b})-, -C(R^{c})(R^{c})-, -C(O)-, -N(SiR^{d})-, -P(R^{d})-, -P(O)(R^{d})-, -C=C(R^{c})(R^{c})- et -P(OR^{d})-, dans lesquels
■ R^{a} représente un radical alkyle en C₁-C₈,
■ R^{b} représente un radical aryle en C₆-C₁₈,
■ R^{c} représente un atome d'hydrogène ou un radical alkyle en C₁-C₈, aryle en C₆-C₁₈, alcoxy en C₁-C₈, aryloxy en C₆-C₁₈, R^{a}(O)- ou R^{b}(O)- ; et
■ R^{d} représente l'un des radicaux R^{a} et R^{b} ;
• Y représente un atome d'oxygène ou de soufre ; et
• les radicaux R5, identiques ou différents, représentent un radical aryle en C₁-C₁₈ ou alkyle en C₁-C₁₈ ;
• R6 représente un radical alkyle en C₁-C₈ ou aryle en C₆-C₁₈ ;
• Z représente un atome d'oxygène ou d'azote ; et
• n est un nombre entier compris entre 2 et 6 ;
• X¹ est choisi dans le groupe constitué par -O-, -S-, -Si(R^{a})₂-, -Si(OR^{a})₂-, -N(C(O)R^{a})-, -N(R^{b})-, -C(R^{c})(R^{c})-, -C(O)-, -N(SiR^{d})-, -P(R^{d})-, -P(O)(R^{d})-, -C=C(R^{c})(R^{c})- et -P(OR^{d})-, dans lesquels
■ R^{a} représente un radical alkyle en C₁-C₈,
■ R^{b} représente un radical aryle en C₆-C₁₈,
■ R^{c} représente un atome d'hydrogène ou un radical alkyle en C₁-C₈, aryle en C₆-C₁₈, alcoxy en C₁-C₈, aryloxy en C₆-C₁₈, R^{a}(O)- ou R^{b}(O)- ; et
■ R^{d} représente l'un des radicaux R^{a} et R^{b}.

3. Procédé selon la revendication 2, **caractérisé en ce que** la réaction est effectuée en présence de rhodium à une concentration de 1 à 1 000 ppm, en particulier de 10 à 250 ppm, par rapport au mélange réactionnel total.

4. Procédé selon la revendication 3, **caractérisé en ce que** le rapport du rhodium au ligand est compris entre 1:1 et 1:100, en particulier entre 1:1 et 1:20.

5. Procédé selon l'une quelconque des revendications 2 à 4, **caractérisé en ce que** la réaction est effectuée à une température de 10 à 180°C, en particulier de 80 à 140°C.

6. Procédé selon l'une quelconque des revendications 2 à 5, **caractérisé en ce que** la réaction est effectuée sous une pression de 0,1 à 200 bars, en particulier de 1 à 100 bars.

7. Procédé selon l'une quelconque des revendications 2 à 6, **caractérisé en ce que** la réaction est effectuée en présence d'un solvant qui est en particulier choisi dans le groupe constitué par l'éther, CO₂, des hydrocarbures fluorés, le toluène et le benzène.

8. Procédé selon l'une quelconque des revendications 2 à 7, **caractérisé en ce que** la réaction est effectuée en présence d'un solvant aprotique polaire qui est en particulier choisi dans le groupe constitué par DMAC, DMF et NMP.

9. Procédé selon l'une quelconque des revendications 2 à 8, **caractérisé en ce que** la réaction est effectuée en présence d'un aldéhyde linéaire oligomère, en particulier en présence du trimère de l'aldéhyde linéaire à préparer.

10. Procédé selon l'une quelconque des revendications 7 à 9, **caractérisé en ce que** la réaction est effectuée dans un mélange en deux phases composé du solvant et d'eau.

11. Procédé selon l'une quelconque des revendications 2 à 10, **caractérisé en ce que** le rapport CO/H₂ pendant l'hydroformylation est compris entre 1:10 et 10:1, en particulier entre 1:2 et 2:1.
